(19) Europäisches Patentamt

European Patent Office

Office européen des brevets

(11) Publication number: **0 308 893 B1**

# (12) EUROPEAN PATENT SPECIFICATION

(45) Date of publication of patent specification: **05.01.94**   (51) Int. Cl.⁵: **C07C 211/14**, C07C 209/00

(21) Application number: **88115477.7**

(22) Date of filing: **21.09.88**

(54) **Process for producing dioctamethylene triamine.**

(30) Priority: **24.09.87 JP 237271/87**

(43) Date of publication of application:
**29.03.89 Bulletin 89/13**

(45) Publication of the grant of the patent:
**05.01.94 Bulletin 94/01**

(84) Designated Contracting States:
**DE FR IT**

(56) References cited:
EP-A- 0 115 637
EP-A- 0 212 287
DE-A- 2 042 760
JP-B-63 011 346

(73) Proprietor: **MITSUBISHI GAS CHEMICAL COM-PANY, INC.**
**5-2, Marunouchi 2-chome**
**Chiyoda-Ku**
**Tokyo, 100(JP)**

(72) Inventor: **Saito, Masao**
**8-14-5, Higashinarashino**
**Narashino-shi Chiba-ken(JP)**
Inventor: **Tsukahara, Kengo**
**1-5-16, Koyo, Kameda-machi**
**Nakakanbara-gun Niigata-ken(JP)**
Inventor: **Maruyama, Keiji**
**1-1, Koei 3-chome**
**Niigata-shi Niigata-ken(JP)**

(74) Representative: **Henkel, Feiler, Hänzel & Part-ner**
**Möhlstrasse 37**
**D-81675 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 308 893 B1

**Description**

This invention relates to a process for producing 1, 17-diamino-9-azaheptadecane (dioctamethylene triamine) (hereinunder referred to as "triamine") which is useful as an intermediate for agricultural agent.

The triamine may be used as an intermediate for synthesizing guazatin, an agricultural agent which is useful as an agricultural bactericide and has antibacterial properties relative to plant pathogenic bacteria.

It is known to hydrogenate dinitriles in the presence of $NH_3$ and a monoamine using inorganic cobalt catalysts (DE OS 20 42 760). Diethylenetriamine has been prepared by reacting ethylenediamine in the presence of e.g. Raney-Co (European publication No. 115 637 A1).

U.S. Patent No. 4,277,622, European Patent Publication No. 212 287 A1 and Japanese Patent Publications (Kokai) Nos. 60-239442 and 60-239443 disclose a process for producing triamine which comprises dimerizing octamethylene diamine (hereinunder referred to as ("diamine").

U.S. Patent No. 4,277,622 discloses a process for producing triamine which comprises adding an acid catalyst, such as nitric acid, hydrochloric acid, a hydrogen chloride gas, p-toluene sulfonic acid, sulfanilic acid or the like to the diamine and heating the mixture to effect deammoniation. However, this process has the following shortcomings:

(i) Strong acids, such as nitric acid must be used;

(ii) The dimerization of the diamine in the presence of the acid catalyst must be carried out at about 200°C for a period of more than 5 hours;

(iii) Since sodium hydroxide must be used for recovering the resulting triamine from the mixture of the triamine and the acid, large amount of waste liquor was to be discharged.

European publication No. 212 287 A2 discloses a process for producing triamines, e.g. 1,17-diamino-9-azaheptadecane which comprises heating a diamine in the presence of expensive palladium, platinum, rhodium, and ruthenium catalysts.

Japanese Publication No. 60-239442 (= JP-B-63-11346) discloses a process for producing triamine which comprises dimerizing the diamine in the presence of an expensive Raney catalyst and in the absence of any solvent. However, not only does the process necessitate use of the expensive Raney catalyst, but when the diamine is heated in the presence of a Raney catalyst, deammoniation is caused, such as to dimerize the diamine, and because in this process, successive reaction is normally caused, such as trimerization of the diamine, or polymerization thereof. The dimerization of the diamine needs to be carried out while maintaining the degree of conversion of the diamine at a level below 50% in order to prevent a successive reaction of this soil. This inevitably lowers the yield of triamine.

The diamine employed in U.S. Patent No. 4,277,622 and Publication No. 60-239442 is obtained by hydrogenerating suberonitrile. Thus these processes require an extra step for hydrogenerating suberonitrile.

Publication No. 60-239443 discloses a process for producing the triamine which comprises heating suberonitrile alone or the mixture of suberonitrile and the diamine in the presence of a Raney catalyst and a hydrogen gas. However, the process also needs expensive Raney catalyst. When the Raney catalyst contacts air, the activity of the catalyst is lost. In addition, the Raney catalyst comprises nickel-aluminum alloy. Before the Raney catalyst is used, it must be treated with potassium hydroxide to eluate the aluminum, or to reduce the catalyst. Therefore, it is difficult to handle the Raney catalyst industrially.

The present inventors have made wide-ranging researches with a view to producing triamine more effective-ly and have found that if the diamine and suberonitrile are heated in the presence of a cobalt complex catalyst under the pressure of hydrogen, suberonitrile is hydrogenated to form the diamine and at the same time triamine can be obtained in a high yield.

This invention relates to a process for producing dioctamethylene triamine, comprising reacting suberonitrile with octamethylene diamine and hydrogen under pressure and in the presence of a cobalt catalyst, characterized in that

(a) the cobalt catalyst is a complex catalyst obtained by heating dicobalt octacarbonyl together with an aromatic nitrile in a solvent capable of dissolving dicobalt octacarbonyl and aromatic nitriles and having a boiling point of more than 100°C, while the reactants are pressurized in an oxygen-free atmosphere,

(b) the reaction is carried out in a solvent within a temperature range of 120°C to 180°C.

When the diamine and suberonitrile are maintained in the presence of the catalyst under the pressure of hydrogen, it is assumed that the following reactions are caused to proceed:

$$NC(CH_2)_6 CN + 4H_2 \rightarrow H_2N(CH_2)_8 NH_2 \qquad (1)$$

$$NC(CH_2)_6 CN + H_2N(CH_2)_8 NH_2 \rightarrow H_2N(CH_2)_8 NH(CH_2)_8 NH_2 + NH_3 \qquad (2)$$

According to the present invention, triamine is produced through the reaction between the diamine and suberonitrile. As the reaction proceeds, the diamine is consumed, but at the same time diamine is newly formed through the hydrogenation of suberonitrile. It is, therefore, unnecessary to replenish the diamine in the present invention while the reaction is proceeding.

The catalyst employed in the present invention is the one employed for hydrogenating an aromatic nitrile. The present catalyst can be obtained by heating a solution of dicobalt octacarbonyl and an aromatic nitrile in a solvent in the substantial absence of oxygen at $100° - 200°C$.

The present catalysts can be prepared by adapting the following method:

Aromatic nitriles that may be employed for preparing the catalysts include, for example, benzonitrile, phthalonitrile, isophthalonitrile, terephthalonitrile and the like.

Solvents capable of dissolving dicobalt octacarbonyl and aromatic nitriles and having a boiling point of more than $100°C$ can be used as a solvent for preparing the catalysts. Examples of such solvents include hydrocarbons, such as toluene, xylene and mesitylene; amides such as dimethyl formamide and dimethyl acetamide; alcohols, such as benzyl alcohol and methylbenzyl alcohol; ketones, such as dipropyl ketone, dibutyl ketone and cyclohexanone; and esters, such as methyl benzoate, propyl benzoate and isobutyl butylate.

It is preferable for the solution of dicobalt octacarbonyl and an aromatic nitrile in a solvent to be heated at $100° - 200°C$. The heating time should be more than 0.5 hours, and preferably 1 - 3 hours.

When the solution is heated in the presence of oxygen, an active catalyst cannot be obtained. For this reason, the air in the atmosphere in which the heating of the solution is to be conducted should be replaced by nitrogen, argon, helium, methane, hydrogen or carbon monoxide.

The cobalt complex obtained by heating the solution of dicobalt octacarbonyl and an aromatic nitrile in a solvent can be used as it is to serve as a catalyst in the present invention. If necessary, the cobalt complex obtained by separating it from the solvent and then drying it can be used as the catalyst. The cobalt complex once dried is stable in air, so it can be handled in the atmosphere. However, the heat-drying of the wet cobalt complex must be carried out in the absence of oxygen. The dried cobalt complex can be used alone as the catalyst, or it can be blended with diatomaceous earth, followed by molding of the mixture. It is unnecessary to reduce the cobalt complex with hydrogen.

The triamine can be prepared by mixing the diamine with suberonitrile and the catalyst and then heating the mixture under the pressure of hydrogen with stirring.

The mixture may be pressurized by hydrogen to $30 - 300 \text{ kg/cm}^2$ and preferably $50 - 200 \text{ kg/cm}^2$. If the mixture is pressurized by hydrogen to a pressure of less than $30 \text{ kg/cm}^2$, the reactivity is low, due to insufficient hydrogeneration of suberonitrile. Though a pressure of more than $300 \text{ kg/cm}^2$ is usable, it adds unnecessarily to the cost.

The hydrogen gas employed for pressurizing the mixture may contain nitrogen, methane and another inert gas. In this case, the partial pressure of hydrogen must be within the above range.

The reaction temperature is carried out in the range of $120° - 180°C$ and preferably $130° - 170°C$. High boiling point condensates such as polyamine are likely to be formed at temperatures above $180°C$, and the reactivity of the diamine and the yield of triamine are lowered at temperatures below $120°C$.

The reaction time may be in the range of 0.5 - 5 hours, and preferably 1 - 3 hours. If the reaction time is too short, the reaction and yield are lowered. If it is too long, an excessive amount of polyamine is formed.

According to the present invention, since a specific cobalt complex is used, triamine can be effectively produced from suberonitrile. The advantages of this invention are as follows:

(1) The triamine can be produced through the reaction between the diamine and suberonitrile. As the reaction proceeds, the diamine is consumed and it is newly formed through the hydrogenation of suberonitrile. It is, therefore, unnecessary to replenish the diamine in the reaction system. In addition, it is also unnecessary to hydrogenate suberonitrile in a separate step.

(2) Use of an expensive Raney catalyst is unnecessary. Since no strong acid is used, treatment of waste liquor becomes unnecessary.

Therefore, the cost of production is low. The industrial significance of this invention is very high.

The present invention is further illustrated by the following non-limiting Examples and Comparative runs.

All percentages and parts in these Examples and Comparative runs are by weight, unless otherwise specified.

The yield of the triamine in these examples is calculated in the following way:

$$\text{Yield of triamine} = \frac{\text{Mol of triamine formed x 2}}{\text{Mol of suberonitrile charged}} \times 100$$

### Example 1

Into a 500 mℓ flask equipped with a reflux condenser and an inlet for gas were charged 11.4 g of dicobalt octacarbonyl, 12.5 g of isophthalonitrile and 300 mℓ of meta-xylene diamine (solvent). The flask was purged with nitrogen by introducing nitrogen into the flask through the inlet for nitrogen. The flask was heated in an oil bath being maintained at 160°C. The flask was kept at the temperature for 90 minutes under the refluxing conditions while passing nitrogen through the flask. The flask was cooled to room temperature to obtain the cobalt complex catalyst.

Into a 500 mℓ stainless steel autoclave were charged suberonitrile (14 g, 0.103 mol) , diamine (21 g, 0.146 mol), mesitylene (100 mℓ: solvent) and the cobalt complex catalyst (15 g) obtained by carrying out thermal treatment of dicobalt octacarbonyl together with isophthalonitrile.

The autoclave was sealed and pressurized by hydrogen to 100 atms. The autoclave was set in a shaking apparatus. The reaction was carried out at 150°C for 2 hours with shaking. After the reaction had been completed, the autoclave was cooled to room temperature and the excess hydrogen was discharged. The catalyst was filtered. The solvent, mesitylene was discharged by an evaporator at a reduced pressure. The residue was distilled at a reduced pressure under an nitrogen atmosphere to obtain triamine (9.5 g). The yield of triamine was 68.0%. The diamine (23 g) was recovered by reducing-distillation as mentioned above.

### Example 2

The procedure of Example 1 was repeated except that the reaction was 1 hour instead of 2 hours. A yield of 5.5 g of triamine was obtained. The yield of triamine was 39.3%. The diamine was recovered in an amount of 28 g.

### Example 3

The procedure of Example 1 was repeated except that the amount of suberonitrile charged was 17.5 g (0.128 mol) and the amount of diamine charged was 17.5 g (0.121 mol).

A yield of 9.5 g of triamine was obtained. The yield of triamine was 54.4% and 22 g of the diamine was recovered.

**Claims**

1. A process for producing dioctamethylene triamine, comprising reacting suberonitrile with octamethylene diamine and hydrogen under pressure and in the presence of a cobalt catalyst, characterized in that

   (a) the cobalt catalyst is a complex catalyst obtained by heating dicobalt octacarbonyl together with an aromatic nitrile in a solvent capable of dissolving dicobalt octacarbonyl and aromatic nitriles and having a boiling point of more than 100°C, while the reactants are pressurized in an oxygen-free atmosphere,

   (b) the reaction is carried out in a solvent within a temperature range of 120°C to 180°C.

2. The process of claim 1, characterized in that the solvent for obtaining the cobalt catalyst is selected from hydrocarbons, aromatic hydrocarbons substituted by amino groups, amides, alcoholes, ketones, and esters.

3. The process of claim 1 or 2, characterized in that heating of the dicobalt octacarbonyl with the aromatic nitrile is carried out in the presence of hydrogen gas.

EP 0 308 893 B1

4. The process of any of claims 1 to 3, characterized in that the reaction pressure produced by hydrogen is in the range of 30 to 300 kg/cm$^2$.

5. The process of any of claims 1 to 4, characterized in that the reaction time is in the range of 0,5 to 5 h.

6. The process of any of claims 1 to 5, characterized in that the solvent and the octamethylene diamine which is produced by the reaction of suberonitrile with hydrogen gas are recovered by reducing-distillation.

**Patentansprüche**

1. Verfahren zur Herstellung von Dioctamethylentriamin durch Umsetzen von Suberonitril mit Octamethylendiamin und Wasserstoff unter Druck und in Gegenwart eines Kobaltkatalysators, dadurch gekennzeichnet, daß

   (a) der Kobaltkatalysator aus einem durch Erwärmen von Dikobaltoctacarbonyl zusammen mit einem aromatischen Nitril in einem zum Auflösen von Dikobaltoctacarbonyl und aromatischen Nitrilen fähigen Lösungsmittel eines Kochpunkts von mehr als 100°C, während die Reaktanten in einer sauerstofffreien Atmosphäre unter Druck gesetzt sind, erhaltenen Komplexkatalysator besteht und
   (b) die Reaktion in einem Lösungsmittel in einem Temperaturbereich von 120°C bis 180°C durchgeführt wird.

2. Verfahren nach Anspruch 1, dadurch gekennzeichnet, daß das Lösungsmittel zur Gewinnung des Kobaltkatalysators aus Kohlenwasserstoffen, durch Aminogruppen substituierten aromatischen Kohlenwasserstoffen, Amiden, Alkoholen, Ketonen und Estern ausgewählt ist.

3. Verfahren nach Anspruch 1 oder 2, dadurch gekennzeichnet, daß das Erwärmen des Dikobaltoctacarbonyls mit dem aromatischen Nitril in Gegenwart von Wasserstoffgas durchgeführt wird.

4. Verfahren nach einem der Ansprüche 1 bis 3, dadurch gekennzeichnet, daß der durch Wasserstoff gebildete Reaktionsdruck im Bereich von 30 - 300 kg/cm$^2$ liegt.

5. Verfahren nach einem der Ansprüche 1 bis 4, dadurch gekenzeichnet, daß die Reaktionszeit im Bereich von 0,5 - 5 h liegt.

6. Verfahren nach einem der Ansprüche 1 bis 5, dadurch gekennzeichnet, daß das Lösungsmittel und das durch die Reaktion von Suberonitril mit Wasserstoffgas gebildete Octamethylendiamin durch Destillation bei vermindertem Druck wiedergewonnen werden.

**Revendications**

1. Procédé de production de dioctaméthylène triamine comprenant la réaction de subéronitrile avec de l'octaméthylène diamine et de l'hydrogène sous pression et en présence d'un catalyseur de cobalt, caractérisé en ce que

   (a) le catalyseur de cobalt est un catalyseur complexe obtenu en chauffant du dicobalt octacarbonyle avec un nitrile aromatique dans un solvant capable de dissoudre le dicobalt octacarbonyle et les nitriles aromatiques et ayant un point d'ébullition de plus de 100°C tandis que les réactifs sont mis sous pression dans une atmosphère sans oxygène,
   (b) la réaction est effectuée dans un solvant à une température comprise entre 120°C et 180°C.

2. Procédé de la revendication 1, caractérisé en ce que le solvant pour obtenir le catalyseur de cobalt est choisi parmi des hydrocarbures, des hydrocarbures aromatiques substitués par des groupes amino, des amides, des alcools, des cétones et des esters.

3. Procédé de la revendication 1 ou 2, caractérisé en ce que le chauffage du dicobalt octacarbonyle avec le nitrile aromatique est effectué en présence d'hydrogène gazeux.

4. Procédé selon l'une quelconque des revendications 1 à 3, caractérisé en ce que la pression de la réaction produite par l'hydrogène est comprise entre 30 et 300 kg/cm$^2$.

5

5. Procédé selon l'une quelconque des revendications 1 à 4, caractérisé en ce que la durée de la réaction est comprise entre 0,5 et 5 heures.

6. Procédé selon l'une quelconque des revendications 1 à 5, caractérisé en ce que le solvant et l'octaméthylène diamine qui est produite par la réaction du subéronitrile avec l'hydrogène gazeux sont récupérés par distillation réductrice.